# EUROPÄISCHE PATENTSCHRIFT

(11) **EP 2 561 816 B1**
(45) Veröffentlichungstag und Bekanntmachung des Hinweises auf die Patenterteilung: **31.08.2022**
(21) Anmeldenummer: 12176339.5
(22) Anmeldetag: 13.07.2012
(51) Int. Cl.: A61B 17/29, A61B 17/00

(54) **Werkzeug für ein mikroinvasiv-chirugisches Instrument**
Tool for a micro-invasive surgical instrument
Outil pour un instrument chirurgical micro-invasif

(30) Priorität: 24.08.2011 DE 102011081464
(43) Veröffentlichungstag der Anmeldung: 27.02.2013
(73) Patentinhaber: Karl Storz SE & Co. KG, 78532 Tuttlingen (DE)
(72) Erfinder: Bacher, Uwe, 78532 Tuttlingen (DE); Kärcher, Daniel, 78532 Tuttlingen (DE); Stefan, Jochen, 78532 Tuttlingen (DE)

(56) Entgegenhaltungen:
- WO-A1-98/34543
- WO-A2-02/07611
- WO-A2-99/03405
- DE-U1- 9 317 535

## Beschreibung

Die vorliegende Erfindung bezieht sich auf einen Schaft für ein mikroinvasiv-chirurgisches Instrument, ein mikroinvasiv-chirurgisches Instrument und dabei insbesondere auf eine Rotierbarkeit eines Werkzeugs am distalen Endabschnitt des Schafts.

Viele mikroinvasiv-chirurgische Instrumente umfassen einen langen und dünnen Schaft, ein Werkzeug am distalen Endabschnitt des Schafts und eine Handhabungseinrichtung am proximalen Endabschnitt des Schafts. Das Werkzeug umfasst beispielsweise eine Fass-, Präparier-, Biopsie- oder andere Zange, eine Schere oder einen Nadelhalter mit mindestens zwei gerade oder gekrümmten Backen, Schneiden oder anderen Maulteilen, von denen mindestens eine bzw. eines bewegbar ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt. Der Schaft enthält (mindestens) eine Übertragungsstange, die in der Regel in einem geschlossenen Kanal im Inneren des Schafts angeordnet ist. Die Handhabungseinrichtung umfasst ein oder mehrere relativ zueinander bewegbare Betätigungseinrichtungen, beispielsweise zwei Griffteile, die medizinisches Personal mit einer Hand relativ zueinander bewegen kann. Der proximale Endabschnitt und der distale Endabschnitt der Übertragungsstange sind so mit der Betätigungseinrichtung bzw. mit dem Werkzeug gekoppelt, dass eine vom medizinischen Personal auf die Betätigungseinrichtungen ausgeübte Kraft oder eine durch medizinisches Personal hervorgerufene relative Bewegung der Betätigungseinrichtungen auf das Werkzeug übertragen werden, beispielsweise um Backen aufeinander zu zu bewegen bzw. zusammenzudrücken.

Bei der Verwendung eines derartigen mikroinvasiv-chirurgischen Instruments werden das Werkzeug und ein Teil des Schafts beispielsweise durch eine natürliche oder künstliche Körperöffnung in einen natürlichen oder künstlichen Hohlraum im Körper eines Patienten eingeführt. Die Entwicklung mikroinvasiver Operationstechniken geht dahin, immer kleinere und vor allem immer weniger Zugänge zu verwenden. Um beispielsweise in der laparoskopischen Chirurgie durch einen einzigen Trokar hindurch mit einem Endoskop und zwei Instrumenten arbeiten zu können, können Instrumente mit gekrümmten Schäften verwendet werden. Ein Instrument mit einem gekrümmten Schaft kann allerdings innerhalb des Zugangs nicht ohne weiteres um seine Längsachse gedreht werden, um die Orientierung des Werkzeugs an seinem distalen Endabschnitt zu verändern.

In der DE 10 2006 038 516 A1 ist ein medizinisches Rohrschaftinstrument beschrieben, bei dem ein Werkzeug 5, ein Schaft 3 und eine Handhabe 2 zur Reinigung voneinander getrennt werden können.

In der DE 10 2008 015 418 A1 ist ein medizinisches Instrument mit einem gekrümmten Schaft beschrieben. Ein Maulteil ist mittels eines Bajonettverschlusses mit einem Schaft lösbar verbunden. Im verbundenen Zustand ist das Maulteil gegenüber dem Schaft drehbar. Der Schaft ist lösbar mit einer Handhabe verbunden. Mittels eines Handrads, das drehfest mit einem äußeren Schaftrohr verbunden ist, kann der gekrümmte Schaft gegenüber der Handhabe gedreht werden. Ein Innenrohr ist mit einem weiteren Handrad an der Handhabe verbunden. Das Instrument kann als unipolares oder bipolares HF-Instrument ausgebildet sein.

In der DE 10 2008 052 623 A1 ist ein chirurgisches Instrument mit einer Mauleinheit, einem Schaft und einer Griffeinheit beschrieben. Die Mauleinheit ist lösbar am Ende eines Schaftrohrs des Schafts befestigt und gegenüber diesem drehbar.

Werkzeug, Schaft und Handhabungseinrichtung eines mikroinvasiv-chirurgischen Instruments sollen ohne Verwendung von Hilfsmitteln voneinander trennbar und miteinander verbindbar bzw. koppelbar sein, um eine einfache und gründliche Reinigung des Instruments zu ermöglichen. Beispielsweise aus der DE 10 2006 038 516 A1 ist es bekannt, das Werkzeug und den distalen Endabschnitt des Schafts so auszubilden, dass das Werkzeug in einer überoffenen Montagestellung am Schaft montiert und demontiert werden kann. Insbesondere bei einer Drehbarkeit bzw. Rotierbarkeit des Werkzeugs gegenüber dem Schaft im gekoppelten Zustand sind jedoch einige Aspekte sowohl der Kopplung des Werkzeugs mit dem Schaft als auch der Kopplung des Schafts mit der Handhabungseinrichtung bislang nicht vollständig befriedigend gelöst.

Die WO1998034543A1 zeigt einen Schaft für ein mikroinvasiv-chirurgisches Instrument mit einem Schaftrohr gezeigt, wobei der Schaft mit einem Werkzeug mechanisch lösbar koppelbar ist und die Kupplungseinrichtung mit dem distalen Endabschnitt des Schaftrohrs mechanisch verbindbar ist.

DE 93 17 535 U1 zeigt wiederum ein mikroinvasiv-chirurgisches Instrument mit einem Schaft mit Schaftrohr und Betätigungsstab, wobei der Schaft mit einem Werkzeug mechanisch lösbar koppelbar ist und zusätzlich eine Nut aufweist, wobei die Kupplungseinrichtung mit dem distalen Endabschnitt des Schaftrohrs mechanisch verbindbar ist. Weiterhin ist eine Rotation der drei Bauteile, Schaftrohr, Betätigungsstab und Werkzeug, relativ zum Handgriff möglich.

Ebenso ist in der WO 02/07611 A2 ist ein Schaft für ein mikroinvasiv-chirurgisches Instrument, das ein Schaftrohr mit proximalem und distalem Endabschnitt gezeigt, wobei der Schaft mit einem Werkzeug mechanisch lösbar koppelbar ist und zusätzlich eine Nut aufweist und die Einrichtung zum Koppeln mit dem distalen Endabschnitt des Schaftrohrs mechanisch verbindbar und eine Rotation der Kupplungseinrichtung relativ zum Schaftrohr um die Umfangsachse des distalen Endabschnitts des Schafts ermöglicht ist.Ausgehend hiervon besteht eine Aufgabe der vorliegenden Erfindung darin, ein verbessertes Werkzeug für ein mikroinvasiv-chirurgisches Instrument und ein verbessertes mikroinvasiv-chirurgisches Instrument zu schaffen, jeweils insb. mit verbesserter Verbindung zwischen Werkzeug und dem restlichen Instrument.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, die Rotierbarkeit eines Werkzeugs eines mikroinvasiv-chirurgischen Instruments am distalen Endabschnitt des Schafts statt am Werkzeug zu implementieren. Dies kann, abhängig von der Ausgestaltung im Detail, eine Reihe von Vorteilen ermöglichen. Insbesondere kann ein Teil der mechanischen Komplexität vom Werkzeug zum Schaft verschoben werden. Dadurch kann das Werkzeug kostengünstiger herstellbar sein. Dies ist vor allem dann vorteilhaft, wenn mit einem oder wenigen Schäften viele verschiedene Werkzeuge alternativ kombiniert werden sollen. Ein weiterer Vorteil kann darin bestehen, dass ein Rotationslager in den in vielen Fällen mechanisch weniger komplexe distale Endabschnitt eines Schafts einfacher integrierbar sein kann als in ein oft mechanisch deutlich komplexeres Werkzeug. Ein weiterer Vorteil kann darin bestehen, dass die Kupplung zwischen Werkzeug und Schaft näher an das distale Ende des Werkzeugs heranrücken kann, wodurch die beim bestimmungsgemäßen Einsatz des Werkzeugs von der Kupplung aufzunehmenden Momente reduziert werden können.

Ein Schaft für ein mikroinvasiv-chirurgisches Instrument umfasst ein Schaftrohr mit einem proximalen Endabschnitt und einem distalen Endabschnitt, eine Kupplungseinrichtung zum lösbaren mechanischen Koppeln des Schafts mit einem Werkzeug und ein Rotationslager, das die Kupplungseinrichtung mit dem distalen Endabschnitt des Schaftrohrs mechanisch verbindet, wobei das Rotationslager ausgebildet ist, um eine Rotation der Kupplungseinrichtung relativ zum Schaftrohr um die Längsachse des distalen Endabschnitts des Schafts zu ermöglichen.

Das Schaftrohr kann direkt oder indirekt mit einer Handhabungseinrichtung mechanisch verbindbar oder verbunden sein. Eine indirekte Verbindung des Schaftrohrs mit einer Handhabungseinrichtung umfasst beispielsweise eine Kupplungseinrichtung, die mit dem Schaftrohr gefügt ist und eine oder mehrere Nuten oder Stege für eine mechanische Verrastung mit der Handhabungseinrichtung aufweist. Ferner kann das Schaftrohr dauerhaft und nicht zerstörungsfrei ohne Verwendung von Werkzeug von der Handhabungseinrichtung trennbar sein. Das Schaftrohr kann mit einer Handhabungseinrichtung so mechanisch verbindbar oder verbunden sein, dass es um seine Längsachse am proximalen Endabschnittrelativ zur Handhabungseinrichtung rotierbar ist.

Das Schaftrohr weist insbesondere einen zentrischen Kanal zum Aufnehmen einer starren oder biegeelastischen Übertragungsstange auf, wobei sich der zentrische Kanal über die gesamte Länge vom proximalen Ende bis zum distalen Ende des Schaftrohrs erstreckt. Sowohl das Schaftrohr als auch der Kanal weisen jeweils insbesondere einen kreisförmigen Querschnitt bzw. einen Querschnitt mit einem kreisförmigen Rand auf.

Die Kupplungseinrichtung ist insbesondere ausgebildet, um sowohl ein mechanisches Koppeln als auch ein Lösen der mechanischen Kopplung ohne Verwendung von Werkzeug und zerstörungsfrei zu ermöglichen. Dadurch kann die Handhabbarkeit des gesamten mikroinvasiv-chirurgischen Instruments durch medizinisches und anderes Personal vor und nach der Verwendung sowie bei der Reinigung und Sterilisation vereinfacht werden.

Die durch das Rotationslager geschaffene mechanische Verbindung zwischen der Kupplungseinrichtung und dem distalen Endabschnitts des Schaftrohres ist insbesondere nicht ohne Werkzeug zerstörungsfrei lösbar. Das Rotationslager ist insbesondere ausgebildet, um ausschließlich eine Rotation der Kupplungseinrichtung relativ zum Schaftrohr um die Längsachse des Schafts - im Fall eines gekrümmten Schafts um die Längsachse des distalen Endabschnitts des Schafts bzw. um die Längsachse des Schafts an dessen distalem Endabschnitt - zu ermöglichen. Dazu ist das Rotationslager so ausgebildet, dass es alle drei translatorischen Freiheitsgrade und zwei von drei rotatorischen Freiheitsgraden - von mechanischem Spiel abgesehen - unterbindet.

Bei einem Schaft, wie er hier beschrieben ist, ist das Rotationslager insbesondere als Radiaxlager ausgebildet.

Ein Radiaxlager ist eine Kombination aus einem Radiallager, das die beiden translatorischen Freiheitsgrade senkrecht zur Längsachse und die rotatorischen Freiheitsgrade um beide zur Längsachse senkrechten Richtungen unterbindet, und einem Axiallager, das den translatorischen Freiheitsgrad parallel zur Längsachse unterbindet. Durch diese Ausgestaltung beschränkt das Rotationslager die Bewegung der Kupplungseinrichtung relativ zum Schaftrohr auf einen einzigen rotatorischen Freiheitsgrad.

Bei einem Schaft, wie er hier beschrieben ist, kann das Rotationslager einen nach radial innen ragenden Kragen und einen nach radial außen ragenden Kragen umfassen, wobei entweder der nach radial außen ragende Kragen mit der Kupplungseinrichtung starr verbunden und der nach radial innen ragende Kragen mit dem distalen Endabschnitt des Schaftrohrs starr verbunden sind oder umgekehrt.

Die beiden Krägen sind insbesondere jeweils kreisringförmig und symmetrisch zur Längsachse des Schafts an dessen distalem Endabschnitt angeordnet. Der nach radial innen ragende Kragen greift in eine nach radial außen sich öffnende Nut mit entsprechendem Querschnitt, dessen eine Flanke durch den nach radial außen ragenden Kragen gebildet wird. Der nach radial außen ragende Kragen greift in eine nach radial innen sich öffnende Nut mit entsprechendem Querschnitt, deren eine Flanke durch den nach radial innen ragenden Kragen gebildet wird. Anders ausgedrückt hintergreifen die Krägen einander so, dass formschlüssig eine spiel- und reibungsarme Rotierbarkeit der beiden Krägen relativ zueinander und damit auch der Kupplungseinrichtung und des distalen Endes des Schaftrohrs relativ zueinander gegeben ist. Dabei können die Krägen in radialer Richtung jeweils eine geringere Ausdehnung aufweisen als in axialer Richtung.

Insbesondere die mit Bezug auf die beigefügten Figuren dargestellten Ausführungsbeispiele zeigen, dass zwei einander in axialer Richtung hintergreifende Krägen ein robustes, spiel- und reibungsarmes Rotationslager bilden können.

Ein Schaft, wie er hier beschrieben ist, ist insbesondere gekrümmt.

Der Schaft kann in einer Ebene gekrümmt sein, wobei die Mittelpunkte aller Querschnitte des Schafts auf einer ebenen Kurve bzw. einer Kurve in einer Ebene liegen. Alternativ kann der Schaft dreidimensional gekrümmt sein, wobei die Mittelpunkte aller Querschnitte des Schafts auf einer Kurve liegen, die nicht eben ist.

Mit der Längsachse des Schafts oder eines anderen Gegenstands ist hier insbesondere die Achse gemeint, zu der der betreffende Gegenstand rotationssymmetrisch oder im Wesentlichen rotationssymmetrisch ist. Im Fall eines gekrümmten oder elastischen Schafts oder anderen Gegenstands beziehen sich diese Aussagen insbesondere auf deren Enden, die insbesondere zumindest abschnittsweise gerade bzw. nicht gekrümmt sind.

Ein Schaft, wie er hier beschrieben ist, ist insbesondere ausgebildet, um mit einem Werkzeug gekoppelt zu werden, das für eine Kopplung mit einem Schaft ohne Rotationslager vorgesehen ist.

Der Schaft ist also insbesondere für eine Kombination bzw. Verwendung mit einem herkömmlichen Werkzeug vorgesehen und ausgebildet, das für eine herkömmliche Verwendung ohne eine Rotierbarkeit vorgesehen ist. Damit ermöglicht der Schaft die Rotierbarkeit, die, wie eingangs beschrieben, für einige Anwendungen wichtig ist, auch bei Verwendung mit herkömmlichen Werkzeugen, die beispielsweise bereits im Bestand einer Klinik oder einer anderen medizinischen Einrichtung vorhanden sind. Der Schaft kann so mit vergleichsweise geringem Aufwand eine erhebliche Verbesserung der Funktionalität mikroinvasiv-chirurgischer Instrumente ermöglichen. Insbesondere ist die Kupplungseinrichtung des Schafts für eine Kopplung mit einem herkömmlichen Werkzeug ausgebildet.

Bei einem Schaft, wie er hier beschrieben ist, ist die Kupplungseinrichtung insbesondere für eine Verriegelbarkeit einer mechanischen Kopplung mit einem Werkzeug ausgebildet.

Dazu weist die Kupplungseinrichtung insbesondere eine axiale Nut oder einen axialen Schlitz auf, in die eine mit einem Werkzeug verbundene Verriegelungseinrichtung eingreifen kann, um eine Kopplung des Werkzeugs mit der Kupplungseinrichtung des Schafts zu verriegeln.

Bei einem Schaft, wie er hier beschrieben ist, weist die Kupplungseinrichtung insbesondere eine Nut oder einen Schlitz mit einem axialen Abschnitt und einem umfänglichen Abschnitt zum Aufnehmen einer Knagge an einem Werkzeug auf.

Ein axialer Abschnitt ist ein in axialer Richtung oder im Wesentlichen in axialer Richtung sich erstreckender Abschnitt. Ein umfänglicher Abschnitt ist ein in umfänglicher Richtung oder im Wesentlichen in umfänglicher Richtung sich erstreckender Abschnitt. Der axiale Abschnitt und der umfängliche Abschnitt sind insbesondere L-förmig angeordnet, so dass das proximale Ende des axialen Abschnitts in ein Ende des umfänglichen Abschnitts übergeht bzw. mit diesem identisch ist. In eine derartigen Nut oder einen derartigen Schlitz kann eine Knagge durch eine axiale und eine nachfolgende rotatorische Bewegung des Werkzeugs relativ zur Kupplungseinrichtung eingesetzt werden. Die Kupplungseinrichtung kann zwei oder mehr Nuten oder Schlitze aufweisen, die insbesondere durch gleiche Winkel voneinander beabstandet sind, um ein Koppeln von Werkzeug und Kupplungseinrichtung zu vereinfachen und die mechanische Robustheit der Kopplung zu verbessern. Ein Schaft, wie er hier beschrieben ist, kann ferner ein Hülsenbauteil mit einem distalen Abschnitt, der die Kupplungseinrichtung bildet, und einen proximalen Abschnitt, der einen Teil des Rotationslagers bildet, aufweisen.

Das Hülsenbauteil kann für eine einfachere Herstellbarkeit mehrere koaxiale Hülsen umfassen. Beispielsweise weist die innere Hülse eine Nut oder einen Schlitz auf, wie sie oben beschrieben sind, und ist mit einer äußeren, koaxialen Hülse gefügt.

Ein mikroinvasiv-chirurgisches Instrument umfasst einen Schaft, wie er hier beschrieben ist, und ein Werkzeug, das mit der Kupplungseinrichtung am distalen Ende des Schafts lösbar mechanisch koppelbar ist.

Ein mikroinvasiv-chirurgisches Instrument, wie es hier beschrieben ist, kann ferner eine Handhabungseinrichtung umfassen, die mit dem proximalen Ende des Schafts koppelbar oder gekoppelt ist, wobei die Handhabungseinrichtung eine Betätigungseinrichtung zum Drehen einer im Schaft angeordneten Übertragungsstange, deren distaler Endabschnitt mit dem Werkzeug gekoppelt ist, aufweist.

Die Betätigungseinrichtung ist insbesondere für ein manuelles oder motorisches Drehen Drehen eines proximalen Endabschnitts oder eines proximalen Bereichs der Übertragungsstange ausgebildet.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiv-chirurgischen Instruments;
- Figur 2: eine schematische Darstellung des mikroinvasiv-chirurgischen Instruments aus Figur 1 in zerlegter Form;
- Figur 3: eine schematische Darstellung eines Schafts mit einem Werkzeug;
- Figur 4: eine weitere schematische Darstellung des Schafts aus Figur 3;
- Figur 5: eine weitere schematische Darstellung des Schafts aus den Figuren 3 und 4;
- Figur 6: eine schematische Darstellung von Kupplungseinrichtungen;
- Figur 7: eine weitere schematische Darstellung der Kupplungseinrichtungen aus Figur 6;
- Figur 8: eine weitere schematische Darstellung der Kupplungseinrichtungen aus den Figuren 6 und 7;
- Figur 9: eine weitere schematische Darstellung der Kupplungseinrichtungen aus den Figuren 6 bis 8.
- Figur 10: eine schematische Darstellung eines Schafts mit einem weiteren Werkzeug;
- Figur 11: eine weitere schematische Darstellung des Schafts aus Figur 10
- Figur 12: eine schematische Darstellung eines weiteren Schafts mit einem Werkzeug;
- Figur 13: eine weitere schematische Darstellung des Schafts aus Figur 12.

### Beschreibung der Ausführungsformen

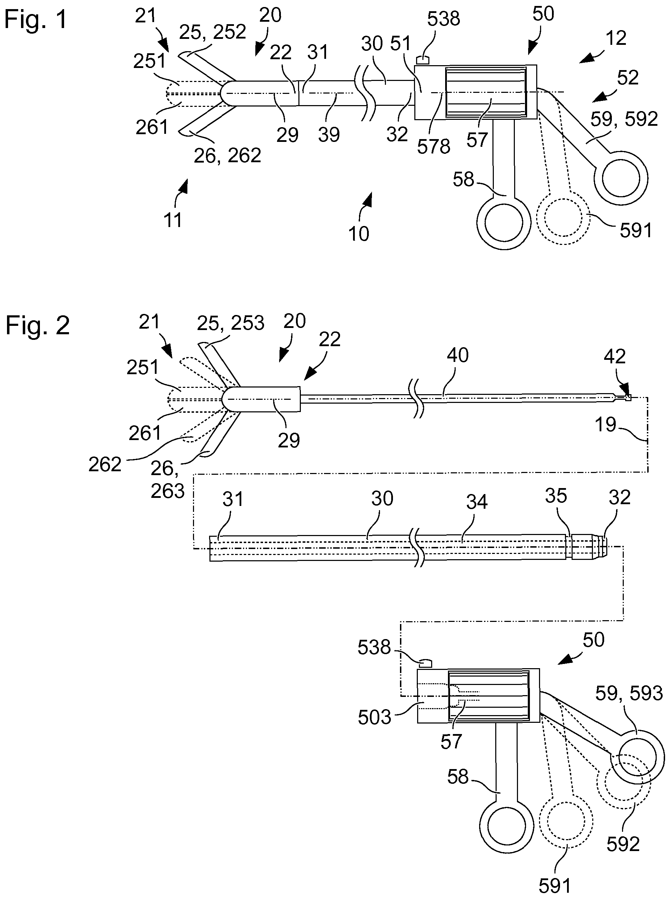

Figur 1 zeigt eine schematische Darstellung eines mikroinvasiv-chirurgischen Instruments 10 mit einem distalen Endabschnitt 11 und einem proximalen Endabschnitt 12. Das mikroinvasiv-chirurgische Instrument 10 umfasst ein Werkzeug 20, einen Schaft 30 und eine Handhabungseinrichtung 50. Am distalen Endabschnitt 21 weist das Werkzeug 20 ein erstes bewegbares Maulteil 25 und ein zweites bewegbares Maulteil 26 auf. Die Maulteile 25, 26 sind in Figur 1 in durchgezogenen Linien in offenen Positionen 252, 262 und in gestrichelten Linien in geschlossenen Positionen 251, 261 dargestellt. Die Maulteile 25, 26 können jeweils gerade oder im Wesentlichen gerade oder in Richtung senkrecht zur Zeichenebene der Figur 1 und/oder - abweichend von der Darstellung in Figur 1 - in der Zeichenebene der Figur 1 gekrümmt sein.

Der proximale Endabschnitt 22 des Werkzeugs 20 ist lösbar mechanisch gekoppelt mit einem distalen Endabschnitt 31 des Schafts 30. Der Schaft 30 ist in Figur 1 stark verkürzt und der Einfachheit halber gerade dargestellt. Abweichend von der Darstellung in Figur 1 kann der Schaft 30 eben oder räumlich gekrümmt sein. Mit einer innerhalb einer Ebene oder - für einige Anwendungen noch vorteilhafter - räumlich gekrümmten Gestalt des Schafts 30 kann das mikroinvasiv-chirurgische Instrument 10 besonders für mikroinvasiv-chirurgische Eingriffe geeignet sein, bei denen ein Endoskop und ein oder mehrere Instrumente gleichzeitig durch einen einzigen Zugang in eine Körperhöhle eingeführt werden.

Der proximale Endabschnitt 32 des Schafts 30 ist mit dem distalen Endabschnitt 51 der Handhabungseinrichtung 50 lösbar mechanisch gekoppelt. Zur Handhabung des mikroinvasiv-chirurgischen Instruments 10 weist die Handhabungseinrichtung 50 ein Drehrad 57, ein erstes Griffteil 58 und ein zweites Griffteil 59 auf. Das Drehrad 57 ist zur Steuerung einer Rotation des Werkzeugs 20, insbesondere der Maulteile 25, 26, um eine Längsachse 29 vorgesehen. Bei dem in Figur 1 dargestellten Beispiel ist das Drehrad 57 um eine Achse 578 drehbar, die gleichzeitig die Längsachse des Schafts 30 an seinem proximalen Endabschnitt 32 ist. Alternativ kann die Achse 578 parallel zur Längsachse des Schafts 30 an seinem proximalen Endabschnitt 32 sein. Ferner weist das Drehrad 57 eine Oberflächenstruktur auf, die auch mit Handschuhen eine zuverlässige Bedienung bzw. Betätigung ermöglicht, beispielsweise die angedeuteten Stege in axialer Richtung. Die Griffteile 58, 59 sind insbesondere - abweichend von der in Figur 1 dargestellten stark stilisierten Gestalt - so angeordnet und geformt, dass medizinisches Personal mit einer Hand beide Griffteile 58, 59 ermüdungsarm greifen und relativ zueinander bewegen kann.

Zumindest eines der beiden Griffteile 58, 59 ist relativ zu den anderen Bestandteilen der Handhabungseinrichtung 50 bewegbar. Bei dem in Figur 1 dargestellten Beispiel sind das erste Griffteil 58 starr und das zweite Griffteil 59 bewegbar angeordnet. Das zweite Griffteil 59 ist insbesondere zwischen einer ersten, in Figur 1 in gestrichelter Linie dargestellten Arbeitsposition 591 und einer zweiten, in Figur 1 in durchgezogener Linie dargestellten Arbeitsposition 592 bewegbar. Das zweite Griffteil 59 der Handhabungseinrichtung 50 ist derart mit den Maulteilen 25, 26 des Werkzeugs 20 mechanisch gekoppelt, dass die Maulteile 25, 26 sich in ihren geschlossenen Positionen 251, 261 befinden, wenn das zweite Griffteil 59 seine erste Arbeitsposition 591 einnimmt, und dass die Maulteile 25, 26 sich in ihren offenen Positionen 252, 262 befinden, wenn das zweite Griffteil 59 seine zweite Arbeitsposition 592 einnimmt.

Figur 2 zeigt eine schematische Darstellung von Bestandteilen bzw. Komponenten des oben anhand der Figur 1 dargestellten mikroinvasiv-chirurgischen Instruments 10, die ohne Verwendung von Werkzeug montiert bzw. zum Instrument zusammengesetzt werden können. Ebenso kann das mikroinvasiv-chirurgische Instrument 10 ohne Werkzeug in die in Figur 2 separat dargestellten Bestandteile bzw. Komponenten zerlegt werden. Durch die die gesamte Figur 2 durchlaufende strichpunktierte Linie 19 ist angedeutet, wie diese Bestandteile bzw. Komponenten zusammenzusetzen sind.

Das Werkzeug 20 ist insbesondere dauerhaft mit einer Übertragungsstange 40 verbunden, die zum Übertragen einer Kraft und eines Drehmoments von der Handhabungseinrichtung 50 zum Werkzeug 20 vorgesehen ist. Der in Figur 2 nicht dargestellte distale Endabschnitt der Übertragungsstange 40 ist derart mit den Maulteilen 25, 26 gekoppelt, dass eine Bewegung der Übertragungsstange 40 parallel zur Längsachse 29 des Werkzeugs 20 eine synchrone Bewegung der Maulteile 25, 26 bewirkt.

Am proximalen Endabschnitt 22 des Werkzeugs 20 und am distalen Endabschnitt 31 des Schafts 30 sind in Figur 2 nicht dargestellte Bajonettkupplungseinrichtungen sowie eine mit der Übertragungsstange 40 gekoppelte Verriegelungseinrichtung vorgesehen. Die Maulteile 25, 26 sind in Figur 2 in durchgezogenen Linien in überoffenen Positionen 253, 263 und in gestrichelten Linien in den bereits oben anhand der Figur 1 beschriebenen geschlossenen und offenen Positionen 251, 252, 261, 262 dargestellt. Wenn die Maulteile 25, 26 sich in den überoffenen Positionen 253, 263 befinden, ist die mit den Maulteilen 25, 26 und dem distalen Endabschnitt der Übertragungsstange 40 gekoppelte und in Figur 2 nicht dargestellte Verriegelungseinrichtung inaktiv. In diesem Zustand können die Übertragungsstange 40 in einen für die Übertragungsstange 40 vorgesehenen Kanal 34 im Schaft 30 eingeführt und der proximale Endabschnitt 22 des Werkzeugs und der distale Endabschnitt 31 des Schafts über die in Figur 2 nicht dargestellten Bajonettkupplungseinrichtungen miteinander lösbar mechanisch verbunden bzw. gekoppelt werden. Ferner kann in diesem entriegelten Zustand eine mechanische Kopplung des proximalen Endabschnitts 22 des Werkzeugs 20 und des distalen Endabschnitts 31 des Schafts 30 über die in Figur 2 nicht dargestellten Bajonettkupplungseinrichtungen gelöst werden.

Wenn die Maulteile 25, 26 sich in den geschlossenen Positionen 251, 261, in den offenen Positionen 252, 262 oder in dazwischen liegenden Positionen befinden, befindet sich die mit dem distalen Endabschnitt der Übertragungsstange 40 und mittelbar mit den Maulteilen 25, 26 gekoppelte Verriegelungseinrichtung in einer Arbeitsposition bzw. in einer Position innerhalb eines Arbeitsbereichs. In der Arbeitsposition bzw. in den Positionen innerhalb des Arbeitsbereichs ist die mechanische Kopplung des proximalen Endabschnitts 22 des Werkzeugs 20 mit dem distalen Endabschnitt 31 des Schafts 30 über die in Figur 2 nicht dargestellten Bajonettkupplungseinrichtungen verriegelt. Wenn die mechanische Verbindung bzw. Kopplung von Werkzeug 20 und Schaft 30 verriegelt ist, können das Werkzeug 20 und der Schaft 30 nicht oder nicht ohne Weiteres zerstörungsfrei voneinander getrennt werden.

Anstelle der Bajonettkupplungseinrichtungen können der proximale Endabschnitt 22 des Werkzeugs 20 und der distale Endabschnitt 31 des Schafts 30 andere Kupplungseinrichtungen aufweisen. Auch in diesem Fall kann eine Verriegelungseinrichtung am Werkzeug 20 vorgesehen sein, die die mechanische Verbindung von Werkzeug 20 und Schaft 30 verriegelt, wenn die Maulteile 25, 26 sich in den überoffenen Positionen 253, 263 befinden.

Wenn die Übertragungsstange 40 in den Kanal 34 des Schafts 30 eingesetzt und der proximale Endabschnitt 22 des Werkzeugs 20 mit dem distalen Endabschnitt 31 des Schafts 30 mechanisch verbunden bzw. gekoppelt ist, kann der proximale Endabschnitt 32 des Schafts 30 mit dem gegenüber dem proximalen Ende des Schafts 30 überstehenden proximalen Endabschnitt 42 der Übertragungsstange 40 in die Handhabungseinrichtung 50 eingesetzt werden. Dazu weist die Handhabungseinrichtung 50 eine in Figur 2 durch eine punktierte Linie angedeutete Ausnehmung 503 auf.

Zum Einsetzen des proximalen Endabschnitts 32 des Schafts 30 und des proximalen Endabschnitts 42 der Übertragungsstange 40 in die Handhabungseinrichtung 50 wird das zweite Griffteil 59 zunächst in eine in Figur 2 in durchgezogener Linie dargestellte Koppelposition 593 gebracht. Wenn sich das zweite Griffteil 59 in der Koppelposition 593 befindet, befindet sich eine in Figur 2 nicht dargestellte Stangenkupplung im Inneren der Handhabungseinrichtung 50 in einer Koppelposition, in der sie den proximalen Endabschnitt 42 der Übertragungsstange 40 aufnehmen oder freigeben kann. Wenn der proximale Endabschnitt 42 der Übertragungsstange 40 ganz in die Handhabungseinrichtung 50 eingeführt ist, wird die in Figur 2 nicht dargestellte Stangenkupplung im Inneren der Handhabungseinrichtung 50 mit dem proximalen Endabschnitt 42 der Übertragungsstange 40 mechanisch verbunden bzw. gekoppelt. Dabei geht das zweite Griffteil 59 abhängig von den Positionen der Maulteile 25, 26 (geschlossene Positionen 251, 261, offene Positionen 252, 262 oder dazwischen) in die erste Arbeitsposition 591, die zweite Arbeitsposition 592 oder eine Position zwischen der ersten Arbeitsposition 591 und der zweiten Arbeitsposition 592 über.

Wenn der proximale Endabschnitt 32 des Schafts 30 ganz in die Handhabungseinrichtung 50 eingeführt ist, greift ein in Figur 2 nicht dargestellter Riegel in eine umlaufende Nut 35 nahe dem proximalen Ende des Schafts 30 ein und verriegelt damit den proximalen Endabschnitt 32 des Schafts 30 in einer vorgesehenen Position in der Handhabungseinrichtung 50. Durch die Verriegelung des proximalen Endabschnitts 32 des Schafts 30 in der Handhabungseinrichtung 50 wird mittelbar auch die mechanische Kopplung des proximalen Endes 42 der Übertragungsstange 40 mit der in Figur 2 nicht dargestellten Stangenkupplung im Innern der Handhabungseinrichtung 50 verriegelt.

Nach Verriegelung des proximalen Endabschnitts 32 des Schafts 30 in der Handhabungseinrichtung 50 und mittelbar des proximalen Endabschnitts 42 der Übertragungsstange 40 in der in Figur 2 nicht dargestellten Stangenkupplung in der Handhabungseinrichtung 50 ist das mikroinvasiv-chirurgische Instrument 10 wie in Figur 1 dargestellt konfiguriert. Durch Bewegung des zweiten Griffteils 59 relativ zum ersten Griffteil 58 zwischen den beiden Arbeitspositionen 591, 592 können die Maulteile 25, 26 zwischen den geschlossenen Positionen 251, 261 und den offenen Positionen 252, 262 bewegt werden. Durch Rotation des Drehrads 57 um die Achse 578 können das Werkzeug 20 und die Maulteile 25, 26, um die Längsachse 29 des Werkzeugs 20 gedreht werden. Dazu ist am bzw. nahe dem distalen Ende des Schafts 30 ein in den Figuren 1 und 2 nicht dargestelltes Rotationslager vorgesehen.

Abweichend von den Darstellungen in den Figuren 1 und 2 kann der Schaft 30 nahe seinem proximalen Ende ein weiteres Drehrad aufweisen, das nahe dem distalen Ende der Handhabungseinrichtung 50 angeordnet ist, wenn der proximale Endabschnitt 32 des Schafts 30 in die Handhabungseinrichtung 50 eingesetzt ist. Mittels dieses in den Figuren 1 und 2 nicht dargestellten Drehrads kann der Schaft 30 um die Längsachse des proximalen Endabschnitts 20 des Schafts 30 gedreht werden. Dies ist insbesondere von Bedeutung, wenn der Schaft 30 abweichend von den Darstellungen in den Figuren 1 und 2 gekrümmt ist. In diesem Fall können der gekrümmte Schaft 30 und das Werkzeug 20 am distalen Ende des gekrümmten Schafts 30 unabhängig voneinander gedreht werden.

Durch Druck auf den Entriegelungsknopf 538 kann der in Figur 2 nicht dargestellte Riegel gegen die Kraft einer Feder verschoben und aus der Nut 35 am Schaft 30 ausgerückt werden. Danach kann der proximale Endabschnitt 32 des Schafts 30 aus der Handhabungseinrichtung 50 entnommen werden. Dabei wird auch die Verriegelung des proximalen Endabschnitts 42 der Übertragungsstange 40 an der in den Figuren 1 und 2 nicht dargestellten Stangenkupplung in der Handhabungseinrichtung 50 gelöst.

Anstelle eines oder - wie in den Figuren 1 und 2 dargestellt - zweier bewegbarer Maulteile 25, 26 kann das Werkzeug 20 eine andere Wirkeinrichtung aufweisen, insbesondere einen Manipulator, beispielsweise einen fingerförmigen Manipulator, oder eine Elektrode, beispielsweise eine hakenförmige Elektrode.

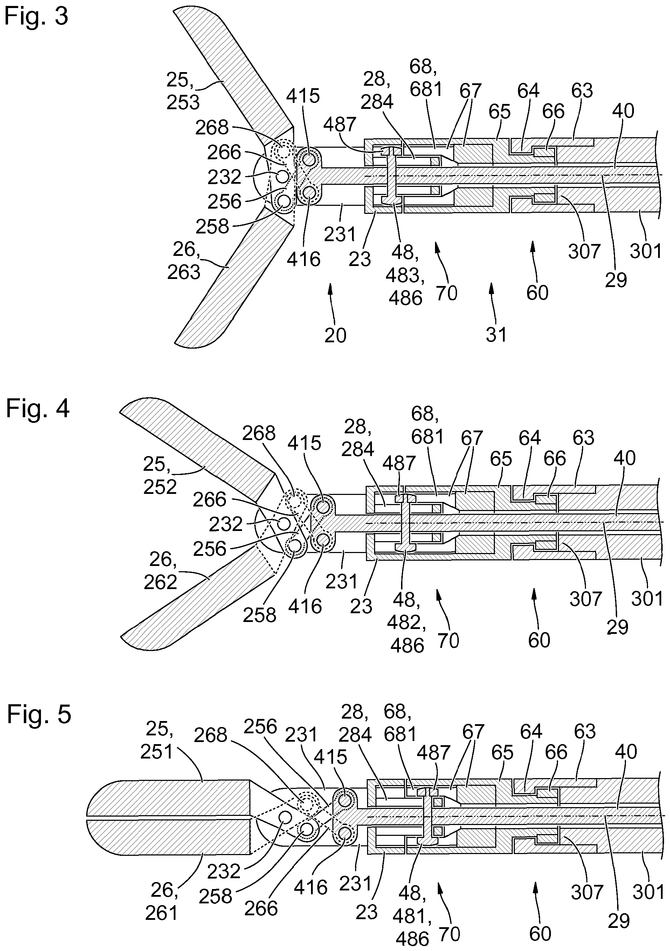

Die Figuren 3 bis 5 zeigen schematische Schnittdarstellungen eines Ausführungsbeispiels des oben anhand der Figuren 1 und 2 dargestellten Werkzeugs 20 und des distalen Endabschnitts des oben anhand der Figuren 1 und 2 dargestellten Schafts 30. Die Schnittebenen der Figuren 3 bis 5 sind parallel zu den Zeichenebenen der Figuren 1 und 2 und enthalten die in den Figuren 1 und 2 angedeutete Längsachse 29 des Werkzeugs 20. In den Figuren 3 bis 5 ist jeweils die Längsachse 29 des Werkzeugs 20 durch eine strichpunktierte Linie angedeutet. Die Längsachse 29 des Werkzeugs 20 ist die Symmetrieachse einiger, jedoch bei weitem nicht aller Merkmale des Werkzeugs 20. Ferner fällt die Längsachse 29 des Werkzeugs mit der Längsachse des Schafts 30 bzw. - wenn der Schaft 30 gekrümmt ist - mit der Längsachse des Schafts an dessen in den Figuren 3 bis 5 dargestelltem distalem Endabschnitt 31 zusammen.

In Figur 3 sind die Maulteile 25, 26 in ihren überoffenen Positionen 253, 263 dargestellt, bei denen die mechanische Verbindung zwischen Werkzeug 20 und Schaft 30 entriegelt ist, d.h. hergestellt oder gelöst werden kann. In Figur 4 sind die Maulteile 25, 26 in ihren offenen Positionen 252, 262 dargestellt, die Verbindung zwischen Werkzeug 20 und Schaft 30 ist verriegelt. In Figur 5 sind die Maulteile 25, 26 in ihren geschlossenen Positionen 251, 261 dargestellt, die Verbindung zwischen Werkzeug 20 und Schaft 30 ist ebenfalls verriegelt.

Die Übertragungsstange 40 weist am distalen Endabschnitt 41 zwei Gelenke 415, 416 auf. Ein erstes Pleuel 256 verbindet das erste Gelenk 415 am distalen Endabschnitt 41 der Übertragungsstange 40 mit einem von einer Welle 232 beabstandeten Gelenk 258 am ersten Maulteil 25. Ein zweites Pleuel 266 verbindet das zweite Gelenk 416 am distalen Endabschnitt 41 der Übertragungsstange 40 mit einem von der Welle 232 beabstandeten Gelenk 268 am zweiten Maulteil 26. In der Zusammenschau der Figuren 3 bis 5 ist erkennbar, dass eine lineare Verschiebung der Übertragungsstange 40 parallel zur Längsachse 29 des Werkzeugs 20 vermittels der Pleuel 256, 266 ein Schwenken der Maulteile 25, 26 um die durch die Welle 232 gebildeten Gelenke bewirkt.

Das Werkzeug 20 weist eine Gelenkeinrichtung 23 auf, die in einem distalen Bereich gabelförmig mit zwei Holmen ausgebildet ist. Ein Holm 231 liegt hinter den Schnittebenen der Figuren 3 bis 5 und ist in den Figuren 3 bis 5 erkennbar. Ein zweiter Holm ist bezüglich der Schnittebenen der Figuren 3 bis 5 symmetrisch zu dem in den Figuren 3 bis 5 gezeigten Holm 231 ausgebildet und angeordnet. Der zweite Holm liegt vor der Schnittebene der Figuren 3 bis 5 und ist deshalb in den Figuren 3 bis 5 nicht dargestellt. Je ein Endabschnitt der senkrecht zu den Schnittebenen der Figuren 3 bis 5 angeordneten Welle 232 ist in einem der beiden Holme 231 der Gelenkeinrichtung 23 gehalten bzw. gelagert.

Der proximale Endabschnitt der Gelenkeinrichtung 23 weist näherungsweise die Gestalt eines Kreiszylindermantels auf, der einen distalen Endabschnitt eines Kupplungsbauteiles 28 umschließt und mit diesem insbesondere stoffschlüssig gefügt ist. Das Kupplungsbauteil 28 weist eine im Wesentlichen zur Längsachse 29 des Werkzeugs 20 rotationssymmetrische Gestalt mit einem zentrischen Kanal, in dem die Übertragungsstange 40 angeordnet ist, auf. Das Kupplungsbauteil 28 weist proximal der Gelenkeinrichtung 23 eine im Wesentlichen kreiszylindrische Gestalt mit einem gegenüber der Gelenkeinrichtung 23 deutlich reduzierten Querschnitt auf.

Abweichend von der Rotationssymmetrie zur Längsachse 29 des Werkzeugs 20 weist das Kupplungsbauteil 28 zwei axiale Schlitze 284 auf, die sich ausgehend vom distalen Ende bis fast zum proximalen Ende des Kupplungsbauteils 28 erstrecken, und in denen die Schnittebenen der Figuren 3 bis 5 liegen. In den Schlitzen 284 ist eine Verriegelungseinrichtung 48 angeordnet, die aus einem Stift 486 und einer ringförmigen Kappe 487 gebildet ist. Ein dünner zylindrischer Abschnitt des Stifts 486 ist in einer Bohrung entsprechenden Querschnitts senkrecht zur Längsachse 29 des Werkzeugs 20 in der Übertragungstange 40 angeordnet. Je ein Endabschnitt des Stifts 486 ragt an gegenüberliegenden Seiten der Übertragungsstange 40 aus dieser hervor und greift in einen der beiden axialen Schlitze 284 im Kupplungsbauteil 28 ein. An einem in den Figuren 3 bis 5 jeweils unten dargestellten Endabschnitt weist der Stift 486 einen vergrößerten Querschnitt auf. Am gegenüberliegenden Endabschnitt ist die Kappe 487 mit entsprechendem Querschnitt an den Stift 486 gefügt.

Ferner weicht die Gestalt des Kupplungsbauteils 28 an dessen proximalem Endabschnitt durch zwei Knaggen von der Rotationssymmetrie ab. Diese Knaggen liegen außerhalb der Schnittebenen der Figuren 3 bis 5 und sind deshalb in diesen Figuren nicht erkennbar.

Bei einer axialen Bewegung der Übertragungsstange 40 zwischen den in den Figuren 3 bis 5 dargestellten Position wird die Verriegelungseinrichtung 48 zwischen der in Figur 3 gezeigten Montageposition 483 und den in den Figuren 4 und 5 gezeigten Arbeitspositionen 482 bzw. 481 bewegt. In der Montageposition 483 der Verriegelungseinrichtung 48 ist diese vollständig innerhalb des zylindermantelförmigen Abschnitts der Gelenkeinrichtung 23 angeordnet. Die Schlitze 284 im Kupplungsbauteil 28 weisen in diesem Bereich an die Endabschnitte der Verriegelungseinrichtung 48 angepasste Querschnitte auf, um diese vollständig aufzunehmen. Bei den in den Figuren 4 und 5 dargestellten Arbeitspositionen 482 bzw. 481 der Verriegelungseinrichtung 48 stehen die beiden Endabschnitte der Verriegelungseinrichtung 48 in einer Richtung senkrecht zur Längsachse 29 des Werkzeugs 20 und parallel zu den Schnittcbcncn der Figuren 3 bis 5 zumindest teilweise gegenüber dem Kupplungsbauteil 28 vor bzw. ragen aus den Schlitzen 284 im Kupplungsbauteil 28 nach außen vor.

Der Schaft 30 umfasst ein Schaftrohr 301 aus Metall oder aus einem anderen Material. An dem in den Figuren 3 bis 5 dargestellten distalen Endabschnitt 31 des Schafts 30 (vgl. Figuren 1 und 2) sind ein Kragenbauteil 63 mit einem nach radial innen ragenden Kragen 64, eine äußere Hülse 65 mit einem nach radial außen ragenden Kragen 66 und eine innere Hülse 67 mit Schlitzen 68 vorgesehen. Die Schlitze 68 in der inneren Hülse 67 weisen jeweils einen in den Figuren 3 bis 5 erkennbaren axialen Abschnitt 681 und einen in den Figuren 3 bis 5 nicht erkennbaren umfänglichen Abschnitt auf.

Das Kragenbauteil 63 ist zu der Längsachse 29 rotationssymmetrisch und weist im Wesentlichen die Gestalt eines Kreiszylindermantels auf. Mit dem kreisringförmigen nach radial innen ragenden Kragen 64 weicht das Kragenbauteil 63 von der Gestalt eines Kreiszylindermantels ab. Der proximale Rand des Kragenbauteils 63 ist mit dem Schaftrohr 301 nahe dessen distalem Ende oder an dessen distalem Endabschnitt 307 gefügt, insbesondere form- und/oder stoffschlüssig gefügt.

Die äußere Hülse 65 ist im Wesentlichen rotationssymmetrisch zur Längsachse 29 des Werkzeugs 20. Ein distaler Bereich der äußeren Hülse 65 weist im Wesentlichen die Gestalt eines Kreiszylindermantels auf, dessen Außendurchmesser den Außendurchmessern des Kragenbauteils 63 und des Schaftrohrs 301 entspricht. Ein proximaler Bereich der äußeren Hülse 65 weist einen reduzierten Querschnitt auf. Der nach radial außen ragende Kragen 66 am proximalen Rand der äußeren Hülse 65 wird bei dem in den Figuren 3 bis 5 gezeigten Beispiel durch ein separat hergestelltes und dann kraft-, form- und/oder stoffschlüssig an die äußere Hülse 65 gefügtes ringförmiges Bauteil gebildet.

Der nach radial innen ragende Kragen 64 des Kragenbauteils 63 ist in einer sich nach radial außen öffnenden kreisringförmigen Nut entsprechenden Durchmessers an der äußeren Hülse 65 angeordnet. Die proximale Flanke dieser Nut wird durch den nach radial außen ragenden Kragen 66 der äußeren Hülse 65 gebildet. Die distale Flanke der sich nach radial außen öffnenden Nut wird durch den stufenförmigen Übergang der äußeren Hülse 65 zu dem erwähnten kreiszylindermantelförmigen distalen Bereich gebildet. Der nach radial außen ragende Kragen 66 an der äußeren Hülse 65 ist in einer Nut entsprechenden Durchmessers zwischen dem distalen Ende des Schaftrohrs 301 und dem nach radial innen ragenden Kragen 64 am Kragenbauteil 63 angeordnet.

Das gegenseitige Hintergreifen des nach radial innen ragenden Kragens 64 am Kragenbauteil 63 und des nach radial außen ragenden Kragens 66 an der äußeren Hülse 65 bzw. die Anordnung der beiden Krägen 64, 66 in Nuten entsprechender Querschnitte schafft eine formschlüssige Verbindung zwischen der äußeren Hülse 65 einerseits und dem Kragenbauteil 63 und dem Schaftrohr 301 andererseits. Diese formschlüssige Verbindung stellt ein Rotationslager - insbesondere ein Radiaxlager - dar, das spiel- und reibungsarm eine Rotation der äußeren Hülse 65 relativ zum Kragenbauteil 63 und zum Schaftrohr 301 um die Längsachse 29 des Werkzeugs 20 zulässt und alle anderen Freiheitsgrade vollständig oder von einem geringen Spiel abgesehen unterbindet.

Die innere Hülse 67 ist im kreiszylindermantelförmigen distalen Bereich der äußeren Hülse 65 angeordnet und mit dieser kraft-, form- und/oder stoffschlüssig gefügt. Die Gestalt des Schlitzes 68 ist an die Gestalt der in den Figuren 3 bis 5 nicht sichtbaren Knaggen am proximalen Endabschnitt des Kupplungsbauteils 28 angepasst, so dass die Knaggen durch die axialen Abschnitte 681 in die umfänglichen Abschnitte der Schlitze 68 in der inneren Hülse 67 eingeführt werden können.

Bei der in Figur 3 dargestellten Montageposition 483 der Verriegelungseinrichtung 48 kann das Werkzeug 20 relativ zu dem durch die äußere Hülse 65 und die innere Hülse 67 gebildeten Hülsenbauteil so weit rotiert werden, wie die Knaggen innerhalb der umfänglichen Abschnitte der Schlitze 68 in der inneren Hülse 67 bewegbar sind. Insbesondere kann das Werkzeug 20 an den distalen Endabschnitt 31 des Schafts 30 angesetzt werden, indem die Knaggen durch eine rein axiale Bewegung parallel zur Längsachse 29 des Werkzeugs 20 in die axialen Abschnitte 681 bis zu den umfänglichen Abschnitten der Schlitze 68 in der inneren Hülse 67 eingeführt werden. Durch eine anschließende Rotation des Werkzeugs 20 relativ zum durch die Hülsen 65, 67 gebildeten Hülsenbauteil um die Längsachse 29 des Werkzeugs 20 werden die Knaggen in von dem axialen Abschnitt 681 beabstandete Bereiche der umfänglichen Abschnitte der Schlitze 68 vcrschobcn.

Wenn die axialen Schlitze 284 im Kupplungsbauteil 28 und die axialen Abschnitte 681 der Schlitze 68 in der inneren Hülse 67, wie in den Figuren 3 bis 5 angedeutet, in einer Ebene liegen, kann durch eine axiale Verschiebung der Übertragungsstange 40 die Verriegelungseinrichtung 48 von der in Figur 3 gezeigten Montageposition 483 in die in den Figuren 4 und 5 gezeigten Arbeitspositionen 482, 481 verschoben werden. Bei den in den Figuren 4 und 5 gezeigten Arbeitspositionen 482, 481 der Verriegelungseinrichtung 48 verhindert Formschluss der Verriegelungseinrichtung 48 mit den Schlitzen 284 im Kupplungsbauteil 28 des Werkzeugs 20 einerseits und mit den axialen Abschnitten 681 der Schlitze 68 in der inneren Hülse 67 andererseits eine relative Rotation des Werkzeugs und des durch die Hülsen 65, 67 gebildeten Hülsenbauteils. Die durch den Formschluss zwischen den Knaggen am Kupplungsbauteil 28 des Werkzeugs 20 einerseits und den umfänglichen Abschnitten der Schlitze 68 in den inneren Hülsen 67 andererseits gebildete mechanische Verbindung zwischen Werkzeug 20 und Schaft 30 ist damit durch die Verriegelungseinrichtung 48 verriegelt. Gleichzeitig ermöglicht das Rotationslager 60 eine Rotation des Werkzeugs 20 relativ zum Schaft 30 um die Längsachse 29 des Werkzeugs 20.

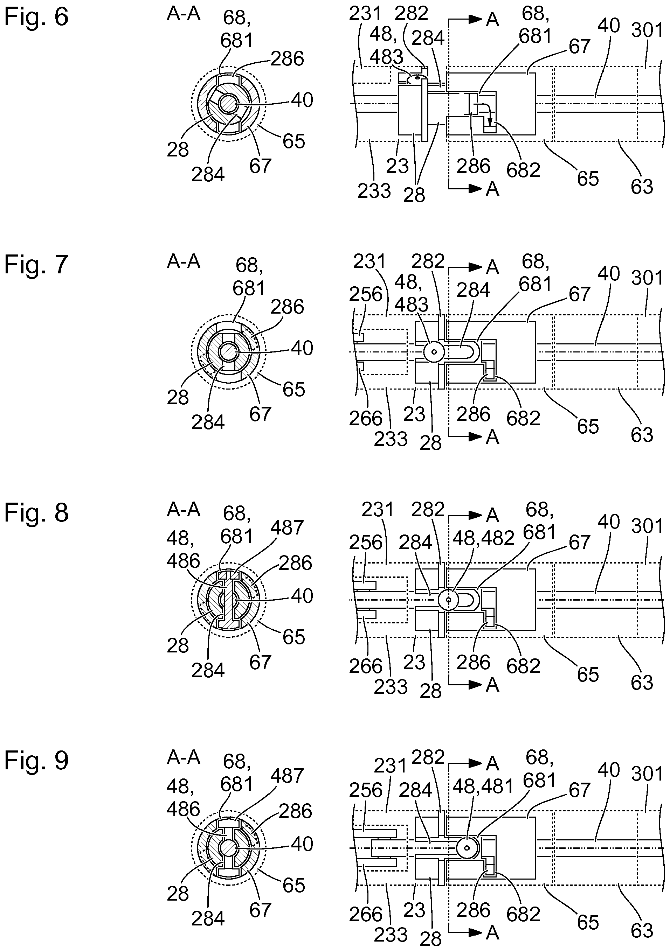

Die Figuren 6 bis 9 zeigen schematische Darstellungen der bereits anhand der Figuren 3 bis 5 dargestellten Kupplungseinrichtung 70 zwischen Werkzeug 20 und Schaft 30. Dabei sind in den Figuren 6 bis 9 jeweils nur das Kupplungsbauteil 28 des Werkzeugs 20, die innere Hülse 67 am distalen Endabschnitt 31 des Schafts 30, die Übertragungsstange 40 und die Verriegelungseinrichtung 48 in durchgezogenen Linien dargestellt. Andere, nicht unmittelbar an der Kupplungseinrichtung 70 bzw. an der Kopplung zwischen Werkzeug 20 und Schaft 30 beteiligte Einrichtungen und Bauteile sind nicht oder nur in gestrichelten Linien dargestellt. Dies gilt insbesondere für die Gelenkeinrichtung 23 des Werkzeugs 20 mit den Holmen 231, 233, das Schaftrohr 301, das Kragenbauteil 63 und die äußere Hülse 65.

In den Figuren 6 bis 9 jeweils links ist ein Schnitt entlang einer Ebene A-A senkrecht zur Längsachse 29 des Werkzeugs 20 (vgl. Figuren 1 bis 5) gezeigt. In den Figuren 6 bis 9 jeweils rechts ist eine Draufsicht gezeigt, deren Zeichenebene jeweils parallel zur Längsachse 29 des Werkzeugs 20 und senkrecht zu den Zeichenebenen der Figuren 1 bis 5 ist.

In Figur 6 ist ein Zwischenzustand während des Ansetzens des Werkzeugs 20 an den Schaft 30 gezeigt, bei dem die Knaggen 286 am Kupplungsbauteil 28 sich in den axialen Abschnitten 681 der L-förmigen Schlitze 681 in der inneren Hülse 67 befinden. Ein Pfeil deutet die Bewegung der Knagge 286 bis zu der in Figur 7 gezeigten Situation an. Durch eine axiale Bewegung des Werkzeugs und des Kupplungsbauteils 28 relativ zur inneren Hülsen 67 und eine anschließende Rotation (bei dem dargestellten Beispiel: um einen Winkel von ca. 60 Grad) werden die Knaggen 286 durch die axialen Abschnitte 681 in die umfänglichen Abschnitte 682 der Schlitze 68 in der inneren Hülse 67 eingeführt bis das Kupplungsbauteil 28 ganz an der inneren Hülse 67 anliegt und die in Figur 7 gezeigte Situation vorliegt.

Die in Figur 7 gezeigte Situation ist die auch in Figur 3 gezeigte Situation, bei der die Verriegelungseinrichtung 48 sich in ihrer Montageposition 483 befindet. Bei den in den Figuren 7 bis 9 gezeigten Situation liegen die Schlitze 284 im Kopplungsbauteil 28 und die axialen Abschnitte 681 der Schlitze 68 in der inneren Hülse 67 in einer Ebene. Die Verriegelungseinrichtung 48 kann deshalb frei durch axiale Bewegung der Übertragungsstange 40 zwischen der in den Figuren 3 und 7 dargestellten Montageposition 483, der in den Figuren 4 und 8 dargestellten Arbeitsposition 482 und der in den Figuren 5 und 9 dargestellten Arbeitsposition 481 verschoben werden. Insbesondere in den Schnitten entlang den Ebenen A-A in den Figuren 8 und 9 ist erkennbar, wie die Verriegelungseinrichtung 48 in den Arbeitspositionen 482, 481 formschlüssig eine relative Rotation des Kupplungsbauteils 28 und der inneren Hülse 67 unterbindet.

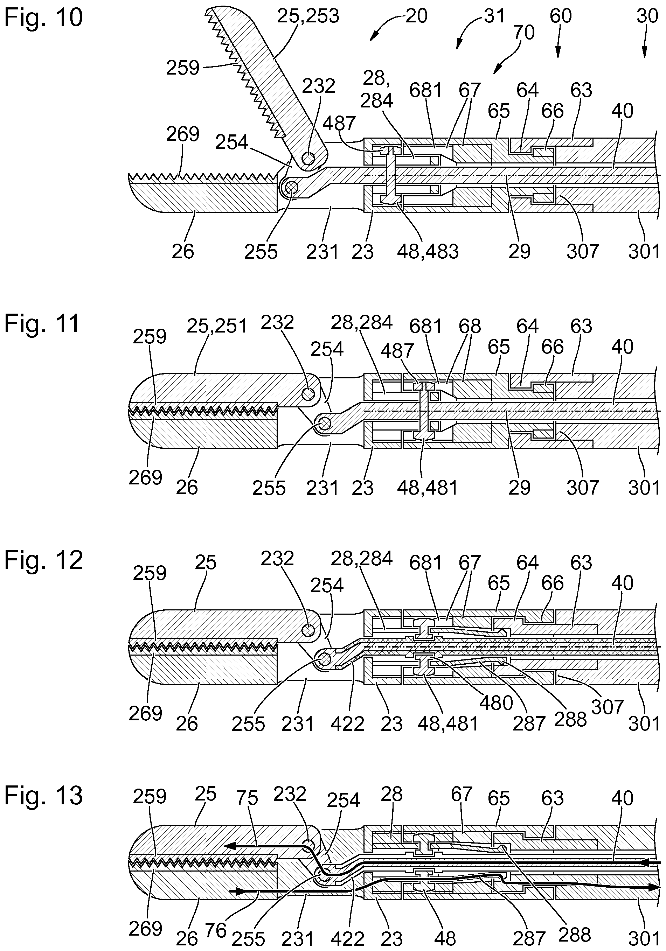

Die Figuren 10 und 11 zeigen schematische Schnittdarstellungen eines weiteren Ausführungsbeispiels des oben anhand der Figuren 1 und 2 dargestellten Werkzeugs 20. Die Schnittebenen der Figuren 10 und 11 sind parallel zu den Zeichenebenen der Figuren 1 und 2, entsprechen den Schnittebenen der Figuren 3 bis 5 und enthalten die Längsachse 29 des Werkzeugs 20. Das Ausführungsbeispiel der Figuren 10 und 11 ähnelt dem oben anhand der Figuren 3 bis 9 dargestellten Ausführungsbeispiel in einigen Merkmalen, insbesondere denen des Rotationslagers 60 und der Kupplungseinrichtung 70, auf deren Beschreibung anhand der Figuren 3 bis 9 verwiesen wird. Das Ausführungsbeispiel der Figuren 10 und 11 unterscheidet sich von dem Ausführungsbeispiel der Figuren 3 bis 9 insbesondere in der Ausgestaltung der Maulteile 25, 26.

Bei dem Ausführungsbeispiel der Figuren 10 und 11 ist nur das erste Maulteil 25 um eine durch eine Welle 232 zwischen den Holmen 231 der Gelenkeinrichtung 23 definierte Achse senkrecht zur Zeichenebene der Figuren 10 und 11 schwenkbar. Das zweite Maulteil 26 ist über die Holme 231 starr mit der Gelenkeinrichtung 23 verbunden, insbesondere einstückig mit der Gelenkeinrichtung 23 ausgebildet. Ein Hebel 254 ist an einem Endabschnitt starr mit dem ersten Maulteil 25 nahe der Welle 232 verbunden und am anderen Endabschnitt über ein Gelenk 255 mit der Übertragungsstange 40 mechanisch gekoppelt. Eine translatorische Bewegung der Übertragungsstange 40 wird durch den Hebel 254 in eine Schwenkbewegung des ersten Maulteils 25 um die durch die Welle 232 definierte Achse übersetzt und umgekehrt. Im Bereich der Gelenkeinrichtung 23 weist die Übertragungsstange 40 eine Biegeflexibilität auf, die erforderlich ist, um eine Bewegung des Gelenks 255 auf einem Kreisbogenabschnitt um die durch die Welle 232 definierte Schwenkachse des ersten Maulteils 25 zu ermöglichen.

In Figur 10 sind das erste Maulteil 25 in einer überoffenen Position 253 und die Verriegelungseinrichtung 48 in einer Montageposition 483 gezeigt. In Figur 11 sind das erste Maulteil 25 in einer geschlossenen Position 251 und die Verriegelungseinrichtung 48 in einer Arbeitsposition 481 gezeigt. Insofern entsprechen einander die Darstellungen der Figuren 3 und 10 und die Darstellungen der Figuren 5 und 11. In der in Figur 10 gezeigten Montageposition 483 der Verriegelungseinrichtung 48 kann das Werkzeug 20 an den distalen Endabschnitt 31 des Schafts 30 angesetzt oder von diesem abgenommen werden. In der in Figur 11 gezeigten Arbeitsposition 481 und in weiteren zwischen der Arbeitsposition 481 und der Montageposition 483 liegenden Arbeitspositionen der Verriegelungseinrichtung 48 ist die mechanische Kopplung zwischen dem Werkzeug 20 und dem distalen Endabschnitt 31 des Schafts 30 in der Kupplungseinrichtung 70 verriegelt.

In allen Positionen des ersten Maulteils 25 und in allen korrespondierenden Positionen der Verriegelungseinrichtung 48 ermöglicht das Rotationslager 60 am distalen Endabschnitt 31 des Schafts 30 eine Rotation der durch die Hülsen 65, 67 gebildete Hülseneinrichtung und des mit ihr gekoppelte Werkzeugs 20 zusammen mit der Übertragungsstange 40 relativ zum Schaftrohr 301 des Schafts 30 um die Längsachse 29 des Werkzeugs 20. Diese Rotation kann insbesondere über das in den Figuren 1 und 2 dargestellte Drehrad 57 an der Handhabungseinrichtung 50 am proximalen Endabschnitt 12 des mikroinvasiv-chirurgischen Instruments 10 angetrieben werden.

An den Maulteilen 25, 26 sind profilierte Hartmetallplatten 259, 269 vorgesehen, die abweichend von der Darstellungen in den Figuren 3 bis 5 auch bei dem dort gezeigten Ausführungsbeispiel vorgesehen sein können. Die Hartmetallplatten 259, 269 können mit ihrer Profilierung dazu beitragen, dass ein von den Maulteilen 25, 26 gefasster Gegenstand nicht entgleiten kann. Die Verwendung eines harten Materials für die Hartmetallplatten 259, 269 reduziert den Verschleiß und ermöglicht - insbesondere im Fall einer Politur der Oberfläche - eine leichte Entfernung von Rückständen bzw. Verunreinigungen.

Die Figuren 12 und 13 zeigen schematische Darstellungen eines weiteren Ausführungsbeispiels des Werkzeugs 20 und des distalen Endabschnitts 31 des Schafts des oben anhand der Figuren 1 und 2 dargestellten mikroinvasiv-chirurgischen Instruments 10. Figur 12 zeigt eine schematische Darstellung eines Schnitts entlang einer Ebene, die parallel zu den Zeichenebenen der Figuren 1 und 2 ist, den Schnittebenen der Figuren 3 bis 5, 10 und 11 entspricht und die Längsachse 29 des Werkzeugs 20 enthält. Figur 13 zeigt eine schematisehe Darstellung, die der Figur 12 ähnelt. Im Unterschied zur Figur 12 sind jedoch in Figur 13 nicht alle Schnittflächen schraffiert und nicht alle schraffierte Flächen Schnittflächen.

Das Ausführungsbeispiel der Figuren 12 und 13 ähnelt in einigen Merkmalen dem Ausführungsbeispiel der Figuren 3 bis 9 und insbesondere dem Ausführungsbeispiel der Figuren 10 und 11. Das Ausführungsbeispiel der Figuren 12 und 13 unterscheidet sich von dem Ausführungsbeispiel der Figuren 10 und 11 insbesondere dadurch, dass am distalen Endabschnitt 307 des Schaftrohrs 301 ein Kragenbauteil 63 angesetzt ist an dessen distalem Endabschnitt ein nach radial außen ragender ringförmiger Kragen 64 vorgesehen ist. Die äußere Hülse 65 weist an ihrem proximalen Rand einen nach radial innen ragenden Kragen 66 auf, der in eine flache ringförmige Nut entsprechenden Querschnitts zwischen dem distalen Ende des Schaftrohrs 301 und dem nach radial außen ragenden Kragen 64 am Kragenbauteil 63 eingreift. Der nach radial außen ragende Kragen 64 am distalen Rand des Kragenbauteils 63 greift in eine ringförmige Nut entsprechenden Querschnitts zwischen dem nach radial innen ragenden Kragen 66 am proximalen Rand der äußeren Hülse 65 und dem proximalen Rand der inneren Hülse 67 ein.

Die Krägen 64, 66 und die Nuten, in die die Krägen 64, 66 eingreifen, sind ähnlich wie bei den Ausführungsbeispielen der Figuren 3 bis 11 hinsichtlich ihrer Querschnitte so aufeinander abgestimmt, dass sie eine spiel- und reibungsarme, formschlüssige Verbindung zwischen dem durch die Hülsen 65, 67 gebildeten Hülsenbauteil und dem Schaftrohr 301 schaffen und gleichzeitig ein Rotationslager bilden. Das genannte Rotationslager ermöglicht eine Rotation des aus den Hülsen 65, 67 gebildeten Bauteils relativ zum Schaftrohr 301 um die Längsachse 29 des Werkzeugs 20.

Das Ausführungsbeispiel der Figuren 12 und 13 unterscheidet sich von den Ausführungsbeispielen der Figuren 3 bis 11 ferner dadurch, dass es für eine bipolare elektrochirurgische Verwendung ausgebildet ist, bei der zwischen den Maulteilen 25, 26 eine elektrische Spannung bzw. ein elektrisches Feld erzeugt werden kann. Dazu weist die Übertragungsstange 40 einen Isoliermantel 422 aus einem elektrisch isolierenden Material auf, der die Übertragungsstange 40 gegenüber dem Schaftrohr 301, dem Kragenbauteil 63, dem Kupplungsbauteil 28 und der Gelenkeinrichtung 23 elektrisch isoliert. Zum Zwecke der elektrischen Isolierung ist die Verriegelungseinrichtung 48 aus einem elektrisch isolierenden Material gefertigt und/oder - wie in den Figuren 12 und 13 angedeutet - mit einem ringförmigen Abschnitt 480 ausgebildet. Der ringförmige Abschnitt 480 umschließt die Übertragungsstange 40 und deren Isoliermantel 422 und ist am Isoliermantel 422 insbesondere durch Kraft-, Form- und/oder Stoffschluss gefügt. Insbesondere ist der ringförmige Abschnitt 480 der Verriegelungseinrichtung 48 durch Formschluss mit dem Isoliermantel 422 relativ zu diesem zumindest in Richtung parallel zur Längsachse 29 des Werkzeugs 20 nicht verschiebbar.

Ferner weist das Kupplungsbauteil 28 proximal Federzungen 287 mit Kontakten 288 auf, die bis zum Kragenbauteil 63 reichen. Die Kontakte 288 werden durch elastische Kräfte der Federzungen 287 an eine korrespondierende kreisringförmige Kontaktfläche am Kragenbauteil 63 gedrückt. Über die Federzungen 287 und die Kontakte 288 sind das Kupplungsbauteil 28 und damit auch die Gelenkeinrichtung 23 und das zweite Maulteil 26 elektrisch leitfähig mit dem Kragenbauteil 63 und über dieses mit dem Schaftrohr 301 verbunden.

Figur 13 zeigt eine schematische Darstellung, die der Darstellung in Figur 12 ähnelt. Anderes als Figur 12 ist Figur 13 jedoch keine reine Schnittdarstellung. In Figur 13 sind nicht alle Schnittflächen schraffiert und nicht alle schraffierte Flächen Schnittflächen. Stattdessen sind der Isoliermantel 422 der Übertragungsstange 40 unschraffiert, das erste Maulteil 25 und alle mit diesem elektrisch leitfähig verbundene Bauteile in Richtung von links unten nach rechts oben schraffiert und das zweite Maulteil 26 und alle mit diesem elektrisch leitfähig verbundene Bauteile von rechts unten nach links oben schraffiert dargestellt. Ferner sind ein Strompfad 75 zum ersten Maulteil 25 und ein Strompfad 76 zum zweiten Maulteil 26 angedeutet, die zur intuitiven Unterscheidung mit Pfeilen in entgegengesetzten Richtungen versehen sind. Der Strompfad 75 zum ersten Maulteil 25 führt über die Übertragungsstange 40, das Gelenk 255 zwischen der Übertragungsstange 40 und dem Hebel 254, den Hebel 254 zum ersten Maulteil 25. Der Strompfad 76 zum zweiten Maulteil 26 führt über das Schaftrohr 301, das Kragenbauteil 63, die Kontakte 288, die Federzungen 287 und weitere Bereiche des Kupplungsbauteils 28, die Gelenkeinrichtung 23 mit den Holmen 231 zum zweiten Maulteil 26. Parallel liegen auch die äußere Hülse 65 und die innere Hülse 67 im Strompfad 76 zum zweiten Maulteil 26.

### Bezugszeichen

- 10: Mikroinvasiv-chirurgisches Instrument
- 11: distaler Endabschnitt des mikroinvasiv-chirurgischen Instruments 10
- 12: proximaler Endabschnitt des mikroinvasiv-chirurgischen Instruments 10
- 19: Richtung des Zusammenbaus
- 20: Werkzeug des mikroinvasiv-chirurgischen Instruments 10
- 21: distaler Endabschnitt des Werkzeugs 20
- 22: proximaler Endabschnitt des Werkzeug 20
- 23: Gelenkeinrichtung
- 231: erster Holm der Gelenkeinrichtung 23
- 232: Welle an der Gelenkeinrichtung 23
- 233: zweiter Holm der Gelenkeinrichtung 23
- 25: erstes Maulteil des Werkzeugs 20
- 251: geschlossene Position des ersten Maulteils 25
- 252: offene Position des ersten Maulteils 25
- 253: überoffene Position des ersten Maulteils 25
- 254: Hebel am ersten Maulteil 25
- 255: Gelenk zwischen Hebel 254 und Übertragungsstange 40
- 256: erstes Pleuel zum ersten Maulteil 25
- 258: Gelenk zwischen dem ersten Maulteil 25 und dem ersten Pleuel 256
- 259: Hartmetallplatte am ersten Maultil 25
- 26: zweites Maulteil des Werkzeugs 20
- 261: geschlossene Position des zweiten Maulteils 26
- 262: offene Position des zweiten Maulteils 26
- 263: überoffene Position des zweiten Maulteils 26
- 266: zweites Pleuel zum zweiten Maulteil 26
- 268: Gelenk zwischen dem zweiten Maulteil 26 und dem zweiten Pleuel 266
- 269: Hartmetallplatte am zweiten Maultil 26
- 28: Kupplungsbauteil
- 282: Kragen am Kupplungsbauteil 28
- 284: axialer Schlitz im Kupplungsbauteil 28
- 286: Knagge am Kupplungsbauteil 28
- 287: Federzunge
- 288: Kontakt
- 29: Längsachse des Werkzeugs 20
- 30: Schaft des mikroinvasiv-chirurgischen Instruments 10
- 301: Schaftrohr des Schafts 30
- 307: distaler Endabschnitt des Metallrohrs 301
- 31: distaler Endabschnitt des Schafts 30
- 32: proximaler Endabschnitt des Schafts 30
- 33: Gelenkeinrichtung am distalen Endabschnitt des Schafts 30
- 34: Kanal im Schaft 30
- 35: umlaufende Nut nahe dem proximalen Endabschnitt 32 des Schafts 30
- 39: Längsachse des Schafts 30 am distalen Endabschnitt 31 des Schafts 30
- 40: Übertragungsstange des mikroinvasiv-chirurgischen Instruments 10
- 41: distaler Endabschnitt der Übertragungsstange 40
- 415: Gelenk zwischen dem distalem Endabschnitt 41 der Übertragungsstange 40 und dem ersten Pleuel 256
- 416: Gelenk zwischen dem distalem Endabschnitt 41 der Übertragungsstange 40 und dem zweiten Pleuel 266
- 42: proximaler Endabschnitt der Übertragungsstange 40
- 422: Isoliermantel an der Übertragungsstange 40
- 48: Verriegelungseinrichtung
- 480: ringförmiger Abschnitt der Verriegelungseinrichtung 48
- 481: erste Arbeitsposition der Verriegelungseinrichtung 48
- 482: zweite Arbeitsposition der Verriegelungseinrichtung 48
- 483: Montageposition der Verriegelungseinrichtung 48
- 486: Stift
- 487: Kappe
- 50: Handhabungseinrichtung des mikroinvasiv-chirurgischen Instruments 10
- 51: distaler Endabschnitt der Handhabungseinrichtung 50
- 52: proximaler Endabschnitt der Handhabungseinrichtung 50
- 538: Entriegelungsknopf
- 57: Drehrad
- 578: Achse
- 58: erstes Griffteil der Handhabungseinrichtung 50
- 59: zweites Griffteil der Handhabungseinrichtung 50
- 591: erste Arbeitsposition des zweiten Griffteils 59
- 592: zweite Arbeitsposition des zweiten Griffteils 59
- 593: Montageposition des zweiten Griffteils 59
- 60: Rotationslager
- 63: Kragenbauteil am distalen Endabschnitt 303 des Metallrohrs 301
- 64: Kragen am Kragenbauteil 63
- 65: äußere Hülse
- 66: Kragen an der äußeren Hülse 65
- 67: innere Hülse
- 68: Schlitz in der inneren Hülse 67
- 681: axialer Abschnitt des Schlitzes 68 in der inneren Hülse 67
- 682: umfänglicher Abschnitt des Schlitzes 68 in der inneren Hülse 67
- 70: Kupplungseinrichtung
- 75: Strompfad zum ersten Maulteil 25
- 76: Strompfad zum zweiten Maulteil 26

## Patentansprüche

1. **Schaft** (30) für ein mikroinvasiv-chirurgisches Instrument (10), mit:
einem **Schaftrohr** (301) mit einem proximalen Endabschnitt (304) und einem distalen Endabschnitt (303);
einer **Kupplungseinrichtung** (70) zum lösbaren mechanischen Koppeln des Schafts (30) mit einem Werkzeug (20) am distalen Endabschnitt (303) des Schafts (30);
wobei die Kupplungseinrichtung (70) eine **Nut** oder einen Schlitz (68) mit einem axialen Abschnitt (681) und einem umfänglichen Abschnitt (682) zum Aufnehmen einer Knagge (286) an einem Werkzeug (20) aufweist, **gekennzeichnet durch** ein **Rotationslager** (60), das die Kupplungseinrichtung (70) mit dem distalen Endabschnitt (303) des Schaftrohrs (301) mechanisch verbindet;
wobei das Rotationslager (60) ausgebildet ist, um eine Rotation der Kupplungseinrichtung (70) relativ zum Schaftrohr (301) **um die Längsachse** (39) des distalen Endabschnitts (31) des Schafts (30) zu ermöglichen.

2. Schaft (30) nach dem vorangehenden Anspruch, bei dem das Rotationslager (60) als **Radiaxlager** ausgebildet ist.

3. Schaft (30) nach einem der vorangehenden Ansprüche, bei dem das **Rotationslager** (60) einen nach radial innen ragenden **Kragen** (64) und einen nach radial außen ragenden **Kragen** (66) umfasst, wobei entweder der nach radial außen ragende Kragen (66) mit der Kupplungseinrichtung (70) starr verbunden und der nach radial innen ragende Kragen (64) mit dem distalen Endabschnitt (303) des Schaftrohrs (301) starr verbunden sind oder umgekehrt.

4. Schaft (30) nach einem der vorangehenden Ansprüche, wobei der Schaft (30) **gekrümmt** ist.

5. Schaft (30) nach einem der vorangehenden Ansprüche, wobei der Schaft (30) ausgebildet ist, um mit einem **Werkzeug** (20) gekoppelt zu werden, das für eine Kopplung mit einem Schaft ohne Rotationslager vorgesehen ist.

6. Schaft (30) nach einem der vorangehenden Ansprüche, bei dem die Kupplungseinrichtung (70) für eine **Verriegelbarkeit** einer mechanische Kopplung mit einem Werkzeug (20) ausgebildet ist.

7. Schaft (30) nach einem der vorangehenden Ansprüche, ferner mit:
einem **Hülsenbauteil** (65, 67), mit einem distalen Abschnitt, der die Kupplungseinrichtung (70) bildet, und einem proximalen Abschnitt, der einen Teil des Rotationslagers (60) bildet.

8. **Mikroinvasiv-chirurgisches Instrument** (10), mit:
einem **Schaft** (30) nach einem der vorangehenden Ansprüche;
einem **Werkzeug** (20), das mit der Kupplungseinrichtung (70) am distalen Endabschnitt (31) des Schafts (30) lösbar mechanisch koppelbar ist.

9. Mikroinvasiv-chirurgisches Instrument (10) nach dem vorangehenden Anspruch, ferner mit:
einer **Handhabungseinrichtung** (50), die mit dem proximalen Endabschnitt (32) des Schafts (30) koppelbar oder gekoppelt ist, wobei die Handhabungseinrichtung (50) eine Betätigungseinrichtung (57) zum Drehen einer im Schaft (30) angeordneten Übertragungsstange (40), deren distaler Endabschnitt (41) mit dem Werkzeug (20) gekoppelt ist, aufweist.

## Claims

1. Shaft (30) for a micro-invasive surgical instrument (10), having:
a shaft tube (301) with a proximal end portion (304) and a distal end portion (303);
a coupling device (70) for releasably mechanically coupling the shaft (30) to a tool (20) on the distal end portion (303) of the shaft (30);
wherein the coupling device (70) has a groove or a slot (68) with an axial portion (681) and with a circumferential portion (682) for receiving a catch (286) on a tool (20), **characterized by**
a rotation bearing (60) which mechanically connects the coupling device (70) to the distal end portion (303) of the shaft tube (301); wherein the rotation bearing (60) is designed to permit rotation of the coupling device (70) relative to the shaft tube (301) about the longitudinal axis (39) of the distal end portion (31) of the shaft (30).

2. Shaft (30) according to the preceding claim, in which the rotation bearing (60) is designed as a radiax bearing.

3. Shaft (30) according to either of the preceding claims, in which the rotation bearing (60) comprises a radially inwardly protruding collar (64) and a radially outwardly protruding collar (66), wherein either the radially outwardly protruding collar (66) is rigidly connected to the coupling device (70) and the radially inwardly protruding collar (64) is rigidly connected to the distal end portion (303) of the shaft tube (301) or vice versa.

4. Shaft (30) according to one of the preceding claims, wherein the shaft (30) is curved.

5. Shaft (30) according to one of the preceding claims, wherein the shaft (30) is designed to be coupled to a tool (20) which is provided for coupling to a shaft without a rotation bearing.

6. Shaft (30) according to one of the preceding claims, in which the coupling device (70) is designed to be able to lock a mechanical coupling to a tool (20).

7. Shaft (30) according to one of the preceding claims, furthermore having:
a sleeve component (65, 67), with a distal portion which forms the coupling device (70) and with a proximal portion which forms part of the rotation bearing (60).

8. Micro-invasive surgical instrument (10), having:
a shaft (30) according to one of the preceding claims;
a tool (20) which is releasably mechanically couplable to the coupling device (70) on the distal end portion (31) of the shaft (30).

9. Micro-invasive surgical instrument (10) according to the preceding claim, furthermore having:
a handling device (50) which is couplable or coupled to the proximal end portion (32) of the shaft (30), the handling device (50) having an actuating device (57) for rotating a transmission rod (40) which is arranged in the shaft (30) and the distal end portion (41) of which is coupled to the tool (20).

## Revendications

1. **Tige** (30) pour un instrument chirurgical micro-invasif (10), comprenant :
un **tube de tige** (301) ayant une section d'extrémité proximale (304) et une section d'extrémité distale (303) ;
un **dispositif de couplage** (70) pour coupler mécaniquement de manière détachable la tige (30) à un outil (20) au niveau de la section d'extrémité distale (303) de la tige (30) ;
le dispositif de couplage (70) présentant une **rainure** ou une fente (68) avec une section axiale (681) et une section circonférentielle (682) pour recevoir un taquet (286) sur un outil (20),
**caractérisé par** un **palier de rotation** (60), qui relie mécaniquement le dispositif de couplage (70) à la section d'extrémité distale (303) du tube de tige (301) ;
le palier de rotation (60) étant configuré pour permettre une rotation du dispositif de couplage (70) par rapport au tube de tige (301) **autour de l'axe longitudinal** (39) de la section d'extrémité distale (31) de la tige (30).

2. Tige (30) selon la revendication précédente, le palier de rotation (60) étant configuré sous forme de **palier radial.**

3. Tige (30) selon l'une quelconque des revendications précédentes, le **palier de rotation** (60) comprenant un **collet** (64) faisant saillie radialement vers l'intérieur et un **collet** (66) faisant saillie radialement vers l'extérieur, le collet (66) faisant saillie radialement vers l'extérieur étant relié de manière rigide au dispositif de couplage (70) et le collet (64) faisant saillie radialement vers l'intérieur étant relié de manière rigide à la section d'extrémité distale (303) du tube de tige (301) ou inversement.

4. Tige (30) selon l'une quelconque des revendications précédentes, la tige (30) étant **courbée.**

5. Tige (30) selon l'une quelconque des revendications précédentes, la tige (30) étant configurée pour être couplée à un **outil** (20), qui est prévu pour être couplé à une tige sans palier de rotation.

6. Tige (30) selon l'une quelconque des revendications précédentes, le dispositif de couplage (70) étant configuré pour **une possibilité de verrouillage** d'un couplage mécanique avec un outil (20).

7. Tige (30) selon l'une quelconque des revendications précédentes, comprenant en outre :
un composant manchon (65, 67), comprenant une section distale qui forme le dispositif de couplage (70), et une section proximale qui forme une partie du palier de rotation (60).

8. **Instrument chirurgical micro-invasif** (10), comprenant :
une **tige** (30) selon l'une quelconque des revendications précédentes ;
un **outil** (20), qui peut être couplé mécaniquement de manière détachable au dispositif de couplage (70) au niveau de la section d'extrémité distale (31) de la tige (30) .

9. Instrument chirurgical micro-invasif (10) selon la revendication précédente, comprenant en outre :
un dispositif de manipulation (50), qui peut être couplé ou est couplé à la section d'extrémité proximale (32) de la tige (30), le dispositif de manipulation (50) présentant un dispositif d'actionnement (57) pour faire tourner une bielle (40) agencée dans la tige (30), dont la section d'extrémité distale (41) est couplée à l'outil (20) .
